# NOUVEAU FASCICULE DE BREVET EUROPEEN

(11) **EP 2 935 375 B2**
(45) Date de publication et mention de la décision concernant l'opposition: **14.04.2021**
(45) Mention de la délivrance du brevet: 05.04.2017
(21) Numéro de dépôt: 13818355.3
(22) Date de dépôt: 19.12.2013
(51) Int. Cl.: C08F 220/28, A61K 8/81

(54) **AGENT POLYMERIQUE POUR OBTENIR UNE COMPOSITION AQUEUSE STABLE COMPRENANT DES PARTICULES EN SUSPENSION**
POLYMERWIRKSTOFF ZUR HERSTELLUNG EINER STABILEN WÄSSRIGEN ZUSAMMENSETZUNG MIT SUSPENDIERTEN TEILCHEN
POLYMERIC AGENT FOR OBTAINING A STABLE AQUEOUS COMPOSITION COMPRISING SUSPENDED PARTICLES

(30) Priorité: 20.12.2012 FR 1262409; 21.12.2012 US 201261740478 P
(43) Date de publication de la demande: 28.10.2015
(73) Titulaire: COATEX, 69730 Genay (FR)
(72) Inventeur: SOUZY, Renaud, 38430 Saint-Jean de Moirans (FR); KENSICHER, Yves, F-69620 Theize (FR); GUERRET, Olivier, F-46170 Pern (FR)
(74) Mandataire: Regimbeau
(86) Numéro de dépôt international: PCT/FR2013/053175
(87) Numéro de publication internationale: WO 2014/096709

(56) Documents cités:
- EP-A1- 0 577 526
- EP-A1- 1 465 932
- WO-A1-2009/090204
- WO-A1-2013/092721
- WO-A2-2011/117427
- FR-A1- 2 872 815
- US-A- 4 529 773
- US-A- 6 140 435
- US-A1- 2007 259 794
- US-A1- 2011 230 387
- US-A1- 2012 230 920
- Form used to codify a monomer of VISCOLAM MAC 10, dated 13.03.2001
- Extract from Final Technical Report of the Res. Project No.
- Form used to approve VISCOLAM MAC 10, dated 29.05.2007
- Extract from the product listing of RITA Corp. published 3/2006
- Extract from the product listing of Biesterfeld Spez. GmbH, published 2008
- Cosmetic formulation of Dow Corning, published 2010
- Cosmetic standard formulary from Dr. Straetmans, published 2008
- IS S2009/0332, published 14.04.2010
- email with VISCOLAM MAC 10 Brochure sent to client 19.02.2006
- email with VISCOLAM MAC 10 Brochure sent to client 06.02.2006
- Technical Data Sheet of VISCOLAM published 06/2006
- ACULYN 88 Technical Data Sheet, published 2007
- 13C-NMR, 1H-NMR and FTIR of ACULYN 88; FTIR of VISCOLAM MAC 10
- ACULYN 88 Brochure, 04.2005
- Product selection Guide from DOW, 03/2012
- ACULYN Rheology Modifiers Brochure, 03/2011
- Lubrizol Pharmaceutical Bulletin 07, 20.10.2008

## Description

La présente invention concerne la formulation de compositions aqueuses stables, comprenant des particules en suspension.

Le problème rencontré lors de la formulation de ces compositions réside essentiellement dans une répartition homogène des particules dans la composition et son maintien dans le temps, notamment au cours du stockage.

Dans la préparation de ces compositions, ce paramètre n'est bien entendu qu'un critère à satisfaire parmi d'autres essentiels, tels que la viscosité, le pH, la clarté, la stabilité.... Et il est donc nécessaire de parvenir à un ajustement optimal de l'ensemble de ces paramètres, et c'est un objectif de l'invention.

Dans la description suivante, il est fait référence à la formulation de compositions cosmétiques mais l'invention n'est pas restreinte à ce domaine d'application et s'étend à tout autre secteur mettant en œuvre de telles compositions, comme celui de la détergence.

Dans son article de la revue Cosmetics & Toiletries® vol. 123, N°12 de décembre 2008, « Formulating at pH 4-5 : How Lower pH Benefits the Skin and Formulations », JW Wiechers s'étonne que le pH naturel de la peau étant acide, d'environ 4,7, la plupart des compositions cosmétiques disponibles aient un pH supérieur, de l'ordre de 6 et plus. De telles variations de pH ne sont pas sans conséquence sur une peau qui y est régulièrement soumise, par exemple en terme de développement de la microflore humaine cutanée, et il met en évidence les bénéfices conférés par des compositions cosmétiques formulées à un pH proche de 4,7. En particulier, il observe une pénétration accrue de certains principes actifs, tout en relevant l'intérêt d'une meilleure conservation de ceux-ci à ces valeurs de pH, ce qui permet de limiter l'usage de conservateurs.

En plus du problème soulevé par la présence de particules, le formulateur de compositions aqueuses acides rencontre des difficultés, car la phase continue de ces compositions n'est pas stable et évolue rapidement vers un déphasage. Ce phénomène est d'autant plus visible quand les compositions comprennent des particules en suspension qui peuvent être elles aussi entrainées dans le fond ou à la surface du contenant.

Le document WO03/061615A décrit des compositions cosmétiques formulées à pH compris entre 6 et 7, utilisées comme fixateur du cheveu. Elles contiennent un épaississant consistant en un polymère dit HASE (hydrophobically modified alkaliswellable) obtenu par polymérisation d'acide méthacrylique, d'acrylate d'éthyle, d'un monomère hydrophobe constitué par une extrémité polymérisable telle que l'acide acrylique, une partie médiane éthoxylée et une extrémité hydrophobe consistant en une chaîne grasse hydrocarbonée, linéaire, et d'un monomère réticulant. Les compositions ainsi formulées possèdent une rhéologie telle, qu'elles sont pulvérisables, qu'elles ne coulent pas sur le cheveu, tout en séchant rapidement une fois appliquées. Ce document s'attache principalement aux propriétés des compositions, lors de leur utilisation.

FR2872815, WO2011/117427 et US 2012/0230920 divulguent des compostions aqueuses comprenant un polymère acrylique conférant des propriétés épaississantes. EP0577526 divulgue des copolymères peignes utilisés comme agent d'antisédimentation de charges minérales et de stabilisation de suspensions aqueuses.

Le problème que l'invention entend résoudre est la mise au point de compositions aqueuses, comprenant une phase continue limpide et des particules en suspension réparties dans la phase continue, le pH de ces compositions étant inférieur à 7 et ces compositions étant stables. Il est effet important que, notamment lors du stockage de telles compositions, les particules soient maintenues en suspension dans la phase continue, celle-ci demeurant limpide. Ces particules ayant une fonction technique ou seulement esthétique doivent pouvoir être visualisées et donc visibles à tout moment. Curieusement, les auteurs ont observé que la viscosité d'une composition apportée par un épaississant et la capacité de ce dernier à maintenir en suspension des particules dans la composition, ne sont pas corrélées.

Selon l'invention, on a découvert un polymère, pour la formulation de compositions aqueuses acides, dans lesquelles ledit polymère agit à la fois, comme épaississant, et clarifiant de la phase continue, tout en permettant une distribution homogène des particules dans la phase continue, lesdites compositions restant stables dans le temps, et en particulier visiblement stables.

La présente invention concerne l'objet des revendications.

Le polymère selon l'invention est obtenu à partir des monomères suivants :
- au moins un monomère A anionique présentant une fonction vinylique polymérisable et un groupement carbonyle, éventuellement sous forme de sel,
- au moins un monomère B non ionique présentant une fonction vynillique polymérisable,
- au moins un monomère C oxyalkylé présentant une fonction vinylique polymérisable et une chaîne hydrocarbonée hydrophobe, et
- au moins un monomère D réticulant.

Selon un mode de réalisation, le monomère C répond à la formule (I) suivante :

T-[(EO)ₙ(PO)_{n'}(BO)_{n"}]-Z (I)

dans laquelle :
- T représente une extrémité permettant la copolymérisation du monomère C,
- [(EO)ₙ(PO)_{n'}(BO)_{n"}] représente une chaîne polyalkoxylée constituée d'unités alkoxylées, réparties en blocs, alternées ou statistiques, choisies parmi les unités éthoxylées EO, les unités propoxylées PO, et les unités butoxylées BO,
- n, n', n" représentant, indépendamment les uns des autres, 0 ou un nombre entier variant de 1 à 150, la somme de n, n' et n" n'étant pas nulle, et
- Z représente une chaîne grasse, linéaire ou ramifiée, d'au moins 16 atomes de carbone.

De manière équivalente, le monomère C peut être représenté de la manière suivante, selon la formule (II) suivante : T-A-Z, dans laquelle :
- T représente une extrémité permettant la copolymérisation du monomère C,
- A représente une chaîne polymérique constituée de :
   - m motifs d'oxyde d'alkylène de formule -CH2CHR1O- avec R1 représentant un groupement alkyle comprenant de 1 à 4 carbones, par exemple un groupement méthyle ou éthyle, et m variant de 0 à 150,
   - p motifs d'oxyde d'alkylène de formule -CH2CHR2O- avec R2 représentant un groupement alkyle comprenant de 1 à 4 carbones, par exemple un groupement méthyle ou éthyle, et p allant de 0 à 150,
   - n motifs d'oxyde d'éthylène avec n variant de 0 à 150, ou de 10, ou 15, à 150, ou de 10, ou 15, à 100, ou de 15 à 50, ou de 15 à 30,
   dans laquelle m+n+p > 0, et
   dans laquelle les motifs d'oxyde d'alkylène de formule -CH2CHR1O-, les motifs d'oxyde d'alkylène de formule -CH2CHR2O- et les motifs d'oxyde d'éthylène sont en bloc, alternés ou statistiques ;
- Z représente une chaîne grasse, linéaire ou ramifiée, d'au moins 16 atomes de carbone.

Selon un mode de réalisation de la présente invention, le polymère est obtenu à partir des monomères suivants :
- au moins un monomère A d'acide acrylique et/ou d'acide méthacrylique et/ou l'un quelconque de leurs sels,
- au moins un monomère B d'acrylates et/ou méthacrylates d'alkyle,
- au moins un monomère C répondant à la formule (I) suivante :

   T-[(EO)ₙ(PO)_{n'}(BO)_{n"}]-Z (I)

   dans laquelle :
   - T représente une extrémité permettant la copolymérisation du monomère C,
   - [(EO)ₙ(PO)_{n'}(BO)_{n"}] représente une chaîne polyalkoxylée constituée d'unités alkoxylées, réparties en blocs, alternées ou statistiques, choisies parmi les unités éthoxylées EO, les unités propoxylées PO, et les unités butoxylées BO,
   - n, n', n" représentant, indépendamment les uns des autres, 0 ou un nombre entier variant de 1 à 150, la somme de n, n' et n" n'étant pas nulle, et
   - Z représente une chaîne grasse, linéaire ou ramifiée, d'au moins 16 atomes de carbone, et
- au moins un monomère D réticulant.

Ainsi, la demande décrit un agent pour l'obtention d'une composition aqueuse, stable, comprenant une phase continue limpide et des particules en suspension réparties dans la phase continue, et ayant un pH inférieur à 7, comprenant un polymère issu d'une polymérisation des monomères A, B, C et D ci-dessus. Elle concerne aussi une composition aqueuse, stable, ainsi préparée.

Avant d'aborder plus en détail l'invention et ses applications, certains termes employés dans la description et les revendications sont ci-après définis.

Une composition de l'invention peut comprendre au moins un ingrédient actif (ou un agent actif) ou un mélange d'ingrédients actifs, sous toute forme que ce soit, et quel que soit le domaine d'application de la composition, comme indiqué précédemment. Le ou les principes actifs peuvent être dissous dans la phase continue de la composition et/ou ils peuvent être sous forme particulaire, non soluble dans la phase continue, et constituer tout ou partie des particules en suspension.

Par « particules à suspendre pour obtenir une composition de l'invention », on entend des corps solides, pleins ou creux, liquides ou gazeux qui peuvent être caractérisés par des formes, des textures, des structures, des compositions, des couleurs et des propriétés finales différentes. A titre indicatif, on peut citer les particules exfoliantes (par exemple les particules de polyéthylène, les coquilles de fruits pillés, les pierres ponce), les particules nourrissantes (par exemple les sphères de collagène), les particules nacrantes (par exemple le mica titane, les glycols distéarate), et les particules esthétiques (par exemple les bulles d'air, les paillettes, les pigments, éventuellement colorés). Pour ce qui est de la suspension de bulles d'air dans la composition, les particules peuvent notamment avoir une taille de 1, 2 ou 3 mm.

Par « alkyle », on comprend un groupe CₘH₂ₘ₊₁, linéaire ou ramifié, où m varie de 1 à 10, de préférence de 1 à 6, voire de 1 à 3 ou 1 à 2. Avantageusement eu égard aux monomères disponibles dans le commerce, il s'agit d'un groupe méthyle ou éthyle.

Par « unités propoxylées PO » et « unités butoxylées BO », on entend des unités éthoxylées porteuses sur l'un ou l'autre de leurs carbones, d'un radical méthyle ou éthyle respectivement. Une unité éthoxylée est une unité -CH₂-CH₂-O.

Par chaîne grasse, on entend une chaîne hydrocarbonée aliphatique d'un acide gras, linéaire ou ramifiée, comprenant au moins 16 atomes de carbone, ou de 16 à 36 atomes de carbone, ou de 16 à 32 atomes de carbone.

Selon l'invention, la clarté ou limpidité d'une composition est mesurée par sa transmittance. Une méthode de détermination de la transmittance est décrite ci-après dans l'exemple 1, Matériels et méthodes. Elle est exprimée en pourcentage et une composition est considérée comme claire ou limpide si elle présente une transmittance d'au moins 40 %.

En plus de la clarté qu'il apporte, l'agent de l'invention permet de maintenir en suspension toute particule présente dans la composition. L'utilisation d'une composition ainsi formulée ne nécessite donc aucune étape de mélange, même si la composition a été stockée plusieurs semaines, voire plusieurs mois.

L'agent de l'invention est particulièrement adapté à la préparation d'une composition ayant un pH inférieur ou égal à 5,5, ou compris entre 4 et 5. De tels pH sont proches de la valeur de pH moyenne de la peau humaine, et il présente ainsi un intérêt majeur en cosmétique.

L'agent ainsi défini répond préférentiellement aux caractéristiques suivantes, considérées seules ou en combinaison :

L'extrémité T représente un radical contenant une fonction insaturée polymérisable, appartenant au groupe des esters acrylique, méthacrylique, maléique, itaconique, ou crotonique. L'extrémité T peut notamment être choisie parmi les groupements acrylates, méthacrylates, allyliques, vinyliques.

Selon un mode de réalisation, le monomère C répond à la formule (III) :

CH₂=C(R1)-COO-[(EO)ₙ(PO)_{n'}(BO)_{n"}]-Z

dans laquelle :
R1 représente H ou CH₃,
n, n', n" et Z ont la même définition que dans la formule (I) ci-dessus.
De manière équivalente, le monomère C répond à la formule (IV) suivante :

CH₂=C(R1)-COO-A-Z

dans laquelle :
R1 représente H ou CH₃,
n, n', n", A et Z ont la même définition que dans la formule (II) ci-dessus.

L'agent selon la présente invention comprend un ou plusieurs monomères réticulants D. Selon un mode de réalisation, il comporte un unique monomère réticulant. Selon un autre mode de réalisation, il comporte deux monomères réticulants. Le ou les monomères réticulants sont utilisés pour générer un copolymère sous forme d'un réseau tridimensionnel.

Selon la présente invention, on utilise un monomère qui est un composé polyinsaturé. Ce composé peut comporter deux, trois ou plusieurs insaturations éthyléniques.

Le monomère réticulant peut avoir un caractère hydrophile, hydrophobe ou amphiphile. Des exemples de ces composés incluent les composés di(méth)acrylate comme le di(méth)acrylate de polyalkylène glycol, notamment le di(méth)acrylate de polypropylène glycol, le di(méth)acrylate d'éthylène glycol, le di(méth)acrylate de polyéthylène glycol, le di(méth)acrylate de triéthylène glycol, le di(méth)acrylate de 1,3-butylène glycol, le 1,6-butylène glycol di(méth)acrylate, le di(méth)acrylate de 1,6-hexanediol, le di(méth)acrylate de neopentyl glycol, le di(méth)acrylate de 1,9-nonanediol, mais aussi le 2,2'-bis(4-(acryloxy-propyloxyphenyl)propane, le 2,2'-bis(4-(acryloxydiethoxy-phényl)propane, et l'acrylate de zinc; les composés tri(méth)acrylate tels que le tri(méth)acrylate de triméthylolpropane, le tri(méth)acrylate triméthylolethane, le tri(méth)acrylate pentaerythritol et le tri(méth)acrylate de tetraméthylolmethane; les composés tetra(méth)acrylate tels que le tetra(méth)acrylate de ditriméthylolpropane, le tetra(méth)acrylate de tetraméthylolmethane, et le tetra(méth)acrylate de pentaerythritol; les composés hexa(méth)acrylate tels que l'hexa(méth)acrylate de dipentaerythritol; les composés penta(méth)acrylate tels que le penta(méth)acrylate de dipentaerythritol; les composés allyl tels que l'allyl (méth)acrylate, le diallylphthalate, l'itaconate de diallyl, le fumarate de diallyl, et le maléate de diallyl; les éthers polyallyl du sucrose ayant de 2 à 8 groupes par molécule, les éthers polyallyl du pentaérythritol tel que le pentaérythritol diallyl éther, le pentaérythritol triallyl éther, et le pentaérythritol tetraallyl éther; les éthers polyallyl du triméthylolpropane tel que l'éther diallyl triméthylolpropane et l'éther triallyl triméthylolpropane. D'autres composés polyinsaturés incluent le divinyl glycol, le divinyl benzène, le divinylcyclohexyl et le méthylènebisacrylamide.

Selon un autre aspect, les monomères réticulants peuvent être préparés par une réaction d'estérification d'un polyol avec un anhydride insaturé tel l'anhydride maléique, ou l'anhydride itaconique, ou par une réaction d'addition avec un isocyanante tel que le 3-isopropenyl-diméthylbenzène isocyanate.

On peut également utiliser les composés insaturés suivants qui réticulent au moyen de leurs groupes carboxyles pendants : polyhaloalkanols tel que le 1 ,3-dichloroisopropanol et le 1,3- dibromoisopropanol; haloépoxyalkanes tel que l'épichlorohydrine, l'épibromohydrin, le 2-méthyl épichlorohydrine, et l'épiiodohydrine; polyglycidyl éthers tels que le 1,4-butanediol diglycidyl éther, glycérine-1,3-diglycidyl éther, ethylene glycol diglycidyl éther, propylène glycol diglycidyl éther, diethylène glycol diglycidyl éther, neopentyl glycol diglycidyl éther, polypropylène glycol diglycidyl éthers, bisphenol A-epichlorohydrin époxy resins et des mélanges.

La proportion des monomères A, B, C et D varie de 10 à 50 %, de 40 à 80 %, de 0,05 à 15 % et de 0,05 à 10 %, respectivement, en poids par rapport au poids total du polymère. Dans un mode de réalisation, la proportion des monomères A, B, C et D varie de 30 à 45 %, de 50 à 65 %, de 0,5 à 12 % et de 1,5 à 5 %, respectivement, en poids par rapport au poids total du polymère.

La préparation des polymères se fait selon des procédés connus de l'homme du métier. Plus précisément, ils sont obtenus par des procédés connus de copolymérisation radicalaire conventionnelle en solution, en émulsion directe ou inverse en bulk, en suspension ou précipitation dans des solvants appropriés, en présence d'initiateurs et d'agents de transfert connus, ou encore par des procédés de polymérisation radicalaire contrôlée tels que la méthode dénommée Reversible Addition Fragmentation Transfer (RAFT), la méthode dénommée Atom Transfer Radical Polymerization (ATRP), la méthode dénommée Nitroxide Mediated Polymerization (NMP) ou encore la méthode dénommée Cobaloxime Mediated Free Radical Polymerization. De préférence, la polymérisation est réalisée en émulsion.

L'invention concerne aussi une composition cosmétique aqueuse, comprenant une phase continue et des particules en suspension dans la phase continue, ladite phase continue et/ou lesdites particules comprenant et/ou consistant en un principe actif cosmétique, et ayant un pH inférieur à 6, avantageusement compris entre 4 et 5, ladite composition comprenant un agent tel que défini précédemment. A titre de principe(s) actif(s), elle peut comprendre une base lavante pour le corps et/ou les cheveux. Dans une telle composition, la proportion de l'agent de l'invention peut varier de 0,1 à 20 %, ou de 5 à 15 %, en poids par rapport au poids total de la composition.

La présente demande décrit encore l'utilisation pour la préparation d'une composition aqueuse stable, comprenant une phase continue limpide et des particules en suspension réparties dans la phase continue, et ayant un pH inférieur à 7, d'un polymère tel que défini précédemment.

La présente invention est maintenant illustrée, de manière non limitative, par les exemples suivants.

### Exemple 1 : Matériels et méthodes

Les avantages de l'invention ressortent de la mesure de propriétés de compositions de l'invention, comparées à celles de compositions comprenant un agent épaississant connu de l'homme du métier.

### Propriétés organoleptiques d'une composition :

On évalue les propriétés organoleptiques de différentes compositions cosmétiques de type gel douche / shampooing formulées et stockées à l'étuve (45°C) pendant 3 mois. L'évaluation est effectuée à température ambiante. Les critères suivants sont pris en compte : Opacité (variation de limpide à opaque, voire blanc intense), Texture (onctueuse, présence de grumeaux, de grains ...), Odeur (apparition ou non d'une odeur, Couleur (variation de l'homogénéité), et Surface (lisse ou non lisse).

### Clarté ou limpidité d'une composition :

La mesure de la limpidité est réalisée par une mesure de la transmittance qui se fait de la manière suivante.

Les mesures sont réalisées sur un Spectromètre UV Genesys 10 UV ™ (Cole Parmer), équipé de cuves Rotilabo-Einmal Kuvetten PS, 4,5 mL. De manière pratique, on préchauffe l'appareil 10 minutes avant utilisation. On réalise d'abord une première mesure au moyen d'une cuve remplie de 3,8 ml d'eau bipermutée (le « blanc »). On réalise ensuite la mesure avec une cuve remplie de 3,8 mL de la solution de composition cosmétique à tester. La transmittance est alors mesurée à la longueur d'onde de 500 nm.

Plus la valeur de transmittance, exprimée en %, est élevée plus la composition cosmétique est limpide. Comme dit précédemment, on estime qu'à une valeur de transmittance d'au moins 40 %, la composition est limpide.

### Viscoélasticité d'une composition :

Des mesures de visco-élasticité de différentes formulations sont réalisées à l'aide d'un rhéomètre de type Haake - RheoStress RS 150. La variation de l'angle de déphasage (δ, en °) en fonction de la contrainte τ (balayage de 0 à 800 Pa) est mesurée à 25°C, grâce au module cône-plan (1°). La valeur limite d'écoulement (YV, Pa ou Dyn/cm²) est déduite de ces mesures.

### Stabilité d'une composition :

Un test de stabilité de différentes formules de protection solaire est réalisé :
- à t = 1 mois - Echantillon stocké à + 4°C
- à t = 3 mois - Echantillon stocké à + 45°C

Les potentielles instabilités telles que le déphasage, le crémage, l'exsudation, le relargage, le dépôt / sédimentation sont observées.

### Viscosité d'une composition :

On mesure la viscosité desdites formulations, à l'aide d'un viscosimètre Brookfield, modèle RVT. Avant la mesure de la viscosité, on laisse chacune des formulations au repos 24 heures à 25°C. Le mobile doit être centré par rapport à l'ouverture du flacon. On mesure ensuite la viscosité à 6 rpm (tours par minute) à l'aide du module approprié. On laisse tourner le viscosimètre jusqu'à ce que la viscosité soit stable.

### Exemple 2 : Shampooing Ultra-Doux Gommant

Cet exemple illustre l'utilisation d'agents selon l'invention dans des formulations cosmétiques de type shampooing ultra-doux, et a pour but de mettre en évidence les propriétés rhéologiques (suspension et viscosité) et organoleptiques apportées selon l'invention.

Ainsi, à partir d'une formulation de shampooing à base de tensioactifs anioniques et zwitterioniques dont la composition figure dans le tableau 1, le but a consisté à vérifier dans cette formule la limpidité, la viscosité et la suspension telles qu'influencées par différents modificateurs de rhéologie dont ceux de l'art antérieur et ceux selon l'invention. Les valeurs mentionnées dans la dernière colonne du tableau indiquent les masses en grammes.

**Tableau 1**

| | |
|---|---|
| 1-Eau DI | QSP 100 |
| 2-Texapon NSO UP, 28 (Cognis) | 32,14 |
| 3-Dehyton PK 45 (Cognis) | 6,67 |
| 4-Modificateur de Rhéologie | **Polymère testé** |
| 5-Hydroxyde de sodium | Qs pH = 6,0 ou 7,0 ± 0,1 |
| 6-Acide lactique | Qs pH 5,0 ± 0.1 |
| 7-Potassium sorbate (Nutrinova) | 0,40 |
| 8-Strawberry Fragrance (Hyteck) | 0,50 |
| 9-Exfoson Quin 300 red, Particules exfoliantes (Soniam) | 2,00 |

Protocole de préparation de la formulation :
- Dans un bécher, on introduit l'eau bipermutée (1), puis sont ajoutés sous agitation les différents ingrédients (2) et (3).
- Après totale homogénéisation, on additionne sous agitation très modérée le modificateur de rhéologie (4).
- On mesure le pH qui est ensuite ajusté à 5,0 ± 0,1 ou 6,0 ± 0,1 ou 7,0 ± 0,1 avec les ingrédients (5) ou (6).
- Après vérification du pH, le conservateur (7) et le parfum (8) sont mélangés sous agitation modérée à la formulation de shampooing.
- On disperse enfin, sous agitation les particules exfoliantes de quinoa (9).

Le tableau 2 récapitule l'ensemble des modificateurs de rhéologie qui ont été utilisés en tant qu'ingrédient (4) dans le cadre des essais du présent exemple 2. On note que leur quantité est exprimée en tant que pourcentage massique par rapport au poids total de la composition. A titre d'exemple, si le pourcentage massique est égal à 5 %, on ajoute 5 g de (4) pour une formulation de 100 g de produit fini.

### Dans le tableau 2 :

REF : Référence / AA : Art Antérieur / INV : Invention / HINV : Hors Invention / NA : non applicable.
Z : nombre d'atomes de carbone de la chaîne grasse, linéaire ou ramifiée.
EDMA : Ethylène Glycol Diméthacrylate
TMP-TMA : Triméthylolpropane Triméthacrylate
NDMA : 1,9-nonanedioldi(méth)acrylate
NPGDE : neopentyl glycol diglycidyl éther

### Essai 1-1 :

L'ingrédient (4) est un sel inorganique : le chlorure de sodium.

### Essai 1-2 :

L'ingrédient, (4) est un additif polymère réticulé du type polyacrylates réticulé, Carbopol® ETD (Lubrizol).

### Essai 1-3 :

L'ingrédient (4) est un additif du type Aqua SF1 (Lubrizol).

### Essai 1-4 :

L'ingrédient (4) est un additif polymère ASE réticulé Acrylate éthyle / Acide méthacrylique.

### Essais 1-5 à 1-14 :

Les ingrédients (4) sont des polymères issus de la polymérisation des monomères A, B, C, D, définis dans le tableau 2, seuls les essais 1-5 à 1-12 correspondant à l'utilisation d'un agent de l'invention.

Les résultats applicatifs sont présentés dans les Tableaux 3 et 4.

**Tableau 2**

| | **Ingrédient (4)** | | | | | | | | | | | |
|---|---|---|---|---|---|---|---|---|---|---|---|---|
| **Essai** | **Etat Physique Solide à 100 % de matière active** | **Etat Physique Solution Aqueuse % de matière active** | **Ingrédient (4) (%)** | **Composition Additif Rhéologique (% massique)** | | | | **Caractéristiques du Monomère C :** | | | | |
| | | | | **Acrylate d'Ethyle B** | **Acide Méthacrylique A** | **Monomère D** | **Monomère C** | **T** | **Carbone Z** | **n'** | **n** | **Ramification Z** |
| 1-1 (REF) | Oui | NA | 3.0 | NA | NA | NA | NA | NA | NA | NA | NA | NA |
| 1-2 (AA) | Oui | NA | 3,0 | NA | NA | NA | NA | NA | NA | NA | NA | NA |
| 1-3 (AA) | Non | 30,0 | 10,0 | NA | NA | NA | NA | NA | NA | NA | NA | NA |
| 1-4 (AA) | Non | 30,0 | 10,0 | 53,5 | 43,1 | 3,4 (EDMA) | 0 | NA | NA | NA | NA | NA |
| 1-5 (INV) | Non | 30,0 | 10,0 | 60,4 | 34,6 | 4,5 (EDMA) | 0,5 | Méthacrylate | 32 | 0 | 25 | ramifié |
| 1-6 (INV) | Non | 30,0 | 8,5 | 58,5 | 39,05 | 0,45 (NDMA) | 2,0 | Méthacrylate | 32 | 0 | 25 | ramifié |
| 1-7 (INV) | Non | 30,0 | 10,0 | 59,6 | 34,1 | 4,4 (EDMA) | 1,9 | Méthacrylate | 16 | 0 | 25 | ramifié |

| **Essai** | **Ingrédient (4)** | | | | | | | | | | | |
|---|---|---|---|---|---|---|---|---|---|---|---|---|
| | **Etat Physique Solide à 100 % de matière active** | **Etat Physique Solution Aqueuse % de matière active** | **Ingrédient (4) (%)** | **Composition Additif Rhéologique (% massique)** | | | | **Caractéristiques du Monomère C :** | | | | |
| | | | | **Acrylate d'Ethyle B** | **Acide Méthacrylique A** | **Monomère D** | **Monomère C** | **T** | **Carbone Z** | **n'** | **n** | **Ramification Z** |
| 1-8 (INV) | Non | 30,0 | 6,6 | 52,8 | 35,5 | 1,6 (EDMA) | 10,1 | Méthacrylate | 20 | 0 | 25 | ramifié |
| 1-9 (INV) | Non | 30,0 | 6,6 | 52,8 | 35,5 | 1,6 (EDMA) | 10,1 | Méthacrylate | 20 | 0 | 36 | ramifié |
| 1-10 (INV) | Non | 30,0 | 7,0 | 57,9 | 37,8 | 0,8 (NPGDE) | 3,5 | Méthacrylate | 20 | 0 | 25 | ramifié |
| 1-11 (INV) | Non | 30,0 | 10,0 | 59,8 | 34,3 | 4,5 (EDMA) | 1,4 | Méthacrylate | 16 | 0 | 25 | linéaire |
| 1-12 (INV) | Non | 30,0 | 10,0 | 52,8 | 42,1 | 2,4 (EDMA) | 2,7 | Méthacrylate | 22 | 0 | 25 | linéaire |
| 1-13 (HINV) | Non | 30,0 | 10,0 | 52,6 | 37,1 | 5,1 (EDMA) | 5,2 | Méthacrylate | 12 | 0 | 23 | linéaire |
| 1-14 (HINV) | Non | 30,0 | 10,0 | 52,6 | 37,1 | 5,1 (TMP-TMA) | 5,2 | Méthacrylate | 12 | 0 | 23 | linéaire |

**Tableau 3**

| Essai | | | | pH 5 | | pH 6 | | pH 7 | |
|---|---|---|---|---|---|---|---|---|---|
| | Viscosité pH5 | Viscosité pH6 | Viscosité pH7 | Transmittance (500 nm) | YV (dyn/cm²) | Transmittance (500 nm) | YV (dyn/cm²) | Transmittance (500 nm) | YV (dyn/cm²) |
| 1-1 | 26900 | 25200 | 17800 | 98,4% | 0 | 98,4% | 0 | 98,6% | 0 |
| 1-2 | 6880 | 9870 | 11700 | 0,5% | 3 | 0,3% | 6 | 0,3% | 8 |
| 1-3 | 5600 | 15220 | 12750 | 5.3% | 35 | 47% | 40 | 90% | 20 |
| 1-4 | 17640 | 18420 | 2075 | 20,4% | 40 | 68,2% | 50 | 39,7% | 4 |
| 1-5 | 4320 | 26600 | 9340 | 57,5% | 20 | 81,5% | 80 | 93,2% | 30 |
| 1-6 | 3500 | 15000 | 21500 | 63,7% | 21 | 75,5% | 29 | 92,8% | 37 |
| 1-7 | 4030 | 25790 | 25600 | 56,8% | 20 | 77,2% | 70 | 99,2% | 60 |
| 1-8 | 3800 | 9950 | 32820 | 94,9% | 10 | 95,3% | 15 | 95,8% | 25 |
| 1-9 | 5100 | 17200 | 18430 | 91,3% | 10 | 93,4% | 20 | 94,4% | 25 |
| 1-10 | 4150 | 12600 | 15450 | 59,5% | 18 | 70,1% | 22 | 92,1% | 31 |
| 1-11 | 4630 | 27800 | 22200 | 44,1% | 15 | 86,1% | 70 | 97,2% | 40 |
| 1-12 | 33100 | 43200 | 36400 | 45,5% | 20 | 72,4% | 50 | 78,9% | 40 |
| 1-13 | 7980 | 9580 | 15420 | 5,3% | 21 | 24% | 79 | 95% | 25 |
| 1-14 | 5430 | 11600 | 14950 | 10,4% | 30 | 25,4% | 35 | 97,3% | 20 |

| | | | | | | | | | |
|---|---|---|---|---|---|---|---|---|---|
| Viscosité : Brookfield 6 rpm (cPs) T₂₄ | | | | | | | | | |

**Tableau 4**

| **Essai n°** | **pH** | **Propriétés Organoleptiques à t = 1 mois** | **Stabilité à t = 1 mois Echantillon stocké à + 4°C** | **Stabilité à t = 3 mois Echantillon stocké à + 45°C** |
|---|---|---|---|---|
| 1-1 | 5 | Etalement : bonne couvrance | Sédimentation complète des particules | Sédimentation complète des particules |
| | | Texture : onctueuse | | |
| | | Odeur : inodorant | | |
| | | Aspect : limpide | | |
| | | Surface : lisse | | |
| 1-2 | 5 | Etalement : bonne couvrance | Stable | Léger dépôt |
| | | Texture : onctueuse | | |
| | | Odeur : inodorant | | |
| | | Aspect : opaque | | |
| | | Surface : lisse | | |
| 1-7 | 5 | Etalement : bonne couvrance | Stable | Stable |
| | | Texture : onctueuse | | |
| | | Odeur : inodorant | | |
| | | Aspect : limpide | | |
| | | Surface : lisse | | |

Le tableau 3 ci-dessus illustre parfaitement la supériorité des polymères selon l'invention par rapport aux polymères de l'art antérieur.

En effet, l'essai 1-1 induit à bas pH des formulations limpides. En revanche, les particules exfoliantes ne sont pas stabilisées. Les essais de l'art antérieur ne sont pas concluants non plus puisque dans cette gamme de pH les formulations sont opaques.

Parallèlement, les tests de stabilité accélérés (Tableau 4) à t = 1 mois (+ 4°C) et à t = 3 mois (+ 45°C) réalisés sur une formulation comportant le polymère de l'essai 1-3 (art antérieur) et en parallèle sur une formulation comportant le polymère de l'essai 1-6 (invention) démontrent une nette différence de stabilité des particules. Les stabilités des particules sont meilleures pour les formulations incorporant les additifs de l'invention.

### Exemple 3 - Gel Douche Exfoliant sans sulfate

Cet exemple concerne la mise en œuvre d'un polymère selon l'invention dans une formulation de gel douche exfoliant qui se caractérise par l'utilisation d'un co-tensioactif « sulfate free » et incorporant les ingrédients suivants (les chiffres dans la dernière colonne indiquent les pourcentages massiques par rapport au poids total de la composition) :

**Tableau 5**

| | |
|---|---|
| 1-Eau DI | QSP 100 |
| 2-Steol® CS 230 (Stepan) | 15,40 |
| 3- Protelan AGL (Z&S) | 2,50 |
| 4-Modificateur de rhéologie | Polymère testé |
| 5-Hydroxyde de sodium | Qs pH = 6,0 ± 0,1 |
| 6-Acide lactique | Qs pH = 6,0 ± 0,1 |
| 7-Geogard 221 (Lonza) | 0,60 |
| 8-Spectrabead 5025 (Micropowder Inc) | 1,00 |
| 9-Floratech Pumice | 1,00 |
| 10-Lemon Fragrance (Fruitaflor Int) | 0,40 |

Protocole de préparation de la formulation :
- Dans un bécher, on introduit l'eau bipermutée (1), puis sont ajoutés sous agitation les différents ingrédients (2) et (3).
- Après totale homogénéisation, on additionne sous agitation très modérée le modificateur de rhéologie (4).
- On mesure le pH qui est ensuite ajusté à 6,0 ± 0,1 avec les ingrédients (5) ou (6).
- Après vérification du pH, le conservateur (7), et le parfum (10) sont mélangés sous agitation modérée à la formulation de shampooing.
- On disperse enfin, sous agitation les particules exfoliantes (8) et (9).

**Tableau 6**

| **Essai** | **Ingrédient (4)** | | | | | | | | | | | |
|---|---|---|---|---|---|---|---|---|---|---|---|---|
| | **Etat Physique Solide à 100 % de matière active** | **Etat Physique Solution Aqueuse % de Matière Active ou poudre** | **Ingrédient (4) (%)** | **Composition Additif Rhéologique (% massique)** | | | | **Caractéristiques du Monomère C** | | | | |
| | | | | **Acrylate d'éthyle B** | **Acide Méthacrylique A** | **Monomère D** | **Monomère C** | **T** | **Z** | **n'** | **n** | **Ramification Z** |
| 2-1 (REF) | Oui | NA | 3,0* | NA | NA | NA | NA | NA | NA | NA | NA | NA |
| 2-2 (AA) | Non | 30,0 | 10,0** | NA | NA | NA | NA | NA | NA | NA | NA | NA |
| 2-3 (INV) | Non | 30,0 | 10,0 | 60,4 | 34,6 | 4,5 (EDMA) | 0,5 | Méthacrylate | 32 | 0 | 25 | ramifié |
| 2-4 (INV) | Non | 30,0 | 8,5 | 58,5 | 39,05 | 0,45 (NDMA) | 2,0 | Méthacrylate | 32 | 0 | 25 | ramifié |
| 2-5 (INV) | Non | 30,0 | 10,0 | 59,6 | 34,1 | 4,4 (EDMA) | 1,9 | Méthacrylate | 16 | 0 | 25 | ramifié |

| | | | | | | | | | | | | |
|---|---|---|---|---|---|---|---|---|---|---|---|---|
| REF : Référence / AA : Art Antérieur / INV : Invention / HINV : Hors Invention / NA : non applicable Essai 2-1 : L'ingrédient (4) est ici un sel inorganique : le chlorure de sodium Essai 2-2 : L'ingrédient (4) est ici un additif polymère réticulé du type acrylates copolymère comme défini dans la présente invention | | | | | | | | | | | | |

**Tableau 7**

| Essais | pH 6 | | |
|---|---|---|---|
| | Viscosité T₂₄ | Transmittance (500 nm) | YV (dyn/cm²) |
| 2-1 | 10 250 | 98,4 % | Pas de suspension |
| 2-2 | 8 430 | 49,1 % | 40 |
| 2-3 | 8 350 | 90,8 % | 40 |
| 2-4 | 8 075 | 87,6 % | 34 |
| 2-5 | 8 180 | 88,2 % | 30 |

Le tableau 7 ci-dessus illustre parfaitement la nette supériorité des polymères selon l'invention par rapport aux polymères de l'art antérieur. La valeur de la transmittance étant nettement plus élevée pour les essais illustrant l'invention. Ceci, en induisant les performances suspensivantes souhaitées.

## Revendications

1. Composition cosmétique aqueuse, comprenant une phase continue et des particules en suspension dans la phase continue, ladite phase continue et/ou lesdites particules comprenant et/ou consistant en un principe actif cosmétique, et ayant un pH inférieur à 6, ladite composition comprenant un agent qui est un polymère constitué :
- d'au moins un monomère A d'acide acrylique et/ou d'acide méthacrylique et/ou de l'un quelconque de leurs sels,
- d'au moins d'un monomère B d'acrylate et/ou de méthacrylate d'alkyle,
- d'au moins un monomère C répondant à la formule (I) suivante :
T-[(EO)ₙ(PO)_{n'}(BO)_{n"}]-Z (I)
dans laquelle :
- T représente une extrémité permettant la copolymérisation du monomère C,
- [(EO)ₙ(PO)_{n'}(BO)_{n"}] une chaîne polyalkoxylée constituée d'unités alkoxylées, réparties en blocs, alternées ou statistiques, choisies parmi les unités éthoxylées EO, les unités propoxylées PO, et les unités butoxylées BO,
- n, n', n" représentant, indépendamment les uns des autres, 0 ou un nombre entier variant de 1 à 150, la somme de n, n' et n" n'étant pas nulle, et
- Z représente une chaîne grasse, linéaire ou ramifiée, d'au moins 16 atomes de carbone,
- d'au moins un monomère D réticulant.

2. Composition selon la revendication 1, selon laquelle ladite composition présente un pH compris entre 4 et 5.

3. Composition selon l'une quelconque des revendications 1 à 2, **caractérisée en ce que**, dans ledit au moins un monomère C répondant à la formule (I) dudit polymère, Z représente une chaîne grasse hydrocarbonée, linéaire ou ramifiée, ayant entre 16 et 32 atomes de carbone.

4. Composition selon l'une quelconque des revendications 1 à 3, **caractérisée en ce que**, dans ledit au moins un monomère C répondant à la formule (I) dudit polymère, T représente une fonction acrylate, méthacrylate, vinyl ou allyl.

5. Composition selon l'une quelconque des revendications 1 à 4, **caractérisé en ce que** le monomère C du dit polymère répond à la formule (III) CH₂=C(R₁)-COO-[(EO)ₙ(PO)_{n'}(BO)_{n"}]-Z dans laquelle R1 représente H ou CH₃.

6. Composition selon l'une quelconque des revendications 1 à 5, **caractérisé en ce que**, le dit polymère contient, par rapport au poids total du polymère :
- de 10 à 50 % en poids de monomères A,
- de 40 à 80 % en poids de monomères B,
- de 0,05 à 15 % en poids de monomères C, et
- de 0,05 à 10 % en poids de monomères D.

7. Composition selon l'une quelconque des revendications 1 à 6, **caractérisée en ce que** la proportion de l'agent varie de 0,1 à 20 % en poids par rapport au poids total de la composition.

## Patentansprüche

1. Wässrige kosmetische Zusammensetzung, umfassend eine kontinuierliche Phase und in der kontinuierlichen Phase suspendierte Teilchen, die kontinuierliche Phase und/oder die suspendierten Teilchen umfassen und/oder bestehen aus einem kosmetischen Wirkstoff, und aufweisen einen pH-Wert von weniger als 6, die Zusammensetzung umfassend ein Mittel, der Mittel ist ein Polymer aus:
- mindestens einem Monomer A von Acrylsäure und/oder Methacrylsäure und/oder einem ihrer Salze,
- mindestens einem Monomer B von Alkylacrylat und/oder -methacrylat,
- mindestens einem Monomer C, das der folgenden Formel (I) entspricht:
T -[(EO)ₙ(PO)_{n'}(BO)_{n"}]-Z (I)
wobei:
- T für eine Endgruppe steht, die die Copolymerisation des Monomers C ermöglicht,
- [(EO)n(PO)n'(BO)n"] für eine polyalkoxylierte Kette aus blockartig, alternierend oder statistisch verteilten Alkoxyleinheiten, die aus Ethoxyleinheiten EO, Propoxyleinheiten PO und Butoxyleinheiten BO ausgewählt sind, steht,
- n, n' und n" unabhängig voneinander für 0 oder eine ganze Zahl im Bereich von 1 bis 150 stehen, wobei die Summe von n, n' und n" nicht gleich null ist, und
- Z für eine lineare oder verzweigte Fettkette mit mindestens 16 Kohlenstoffatomen steht,
- mindestens einem Vernetzungsmonomer D.

2. Zusammensetzung nach Anspruch 1, wobei die Zusammensetzung einen pH-Wert zwischen 4 und 5 aufweist.

3. Zusammensetzung nach einem der Ansprüche 1 bis 2, **dadurch gekennzeichnet, dass** in dem mindestens einen Monomer C, das der Formel (I) entspricht, des Polymers Z für eine lineare oder verzweigte Kohlenwasserstoff-Fettkette mit zwischen 16 und 32 Kohlenstoffatomen steht.

4. Zusammensetzung nach einem der Ansprüche 1 bis 3, **dadurch gekennzeichnet, dass** in dem mindestens einen Monomer C, das der Formel (I) entspricht, des Polymers T für eine Acrylat-, Methacrylat-, Vinyl- oder Allylfunktion steht.

5. Zusammensetzung nach einem der Ansprüche 1 bis 4, **dadurch gekennzeichnet, dass** das Monomer C des Polymers der Formel (III) CH₂=C(R1)-COO-[(EO)ₙ(PO)_{n'}(BO)_{n"}]-Z entspricht, wobei R1 für H oder CH₃ steht.

6. Zusammensetzung nach einem der Ansprüche 1 bis 5, **dadurch gekennzeichnet, dass** das Polymer, bezogen auf das Gesamtgewicht des Polymers:
- 10 bis 50 Gew.-% Monomere A,
- 40 bis 80 Gew.-% Monomere B,
- 0,05 bis 15 Gew.-% Monomere C und
- 0,05 bis 10 Gew.-% Monomere D
enthält.

7. Zusammensetzung nach einem der Ansprüche 1 bis 6, **dadurch gekennzeichnet, dass** der Anteil des Mittels im Bereich von 0,1 bis 20 Gew.-%, bezogen auf das Gesamtgewicht der Zusammensetzung, liegt.

## Claims

1. Aqueous cosmetic composition, comprising a continuous phase and particles in suspension in the continuous phase, said continuous phase and/or said particles comprising and/or consisting of a cosmetic active principle, and having a pH lower than 6, said composition comprising an agent which is a polymer constituted of:
- at least one A monomer of acrylic acid and/or methacrylic acid and/or any of their salts,
- at least one B monomer of alkyl acrylate and/or alkyl methacrylate,
- at least one C monomer corresponding to the following formula (I):
T-[(EO)ₙ(PO)_{n'}(BO)_{n"}]-Z (I)
in which:
- T represents one end enabling the co-polymerization of the C monomer,
- [(EO)ₙ(PO)_{n'}(BO)_{n"}] represents a polyalkoxylated chain consisting of alkoxylated units, distributed into blocks, alternatively or statistically, chosen from among EO ethoxylated units, PO propoxylated units and BO butoxylated units,
- n, n', n" representing, independently of each other, 0 or an integer varying from 1 to 150, the sum of n, n' and n" not being null, and
- Z represents a fatty chain, linear or branched, of at least 16 carbon atoms,
- at least one cross linking monomer D.

2. Composition according to claim 1, wherein the said composition has a pH of between 4 and 5.

3. Composition according to any of claims 1 to 2, **characterized by** the fact that, in the said at least one monomer C of formula (I) of the said polymer, Z represents a hydrocarbonated fatty chain, linear or branched, ranging from 16 to 32 carbon atoms.

4. Composition according to any of claims 1 to 3, **characterized by** the fact that, in the said at least one monomer C of formula (I) of the said polymer, T represents an acrylate, methacrylate, vinyl or allyl function.

5. Composition according to any of claims 1 to 4, **characterized by** the fact that, the monomer C of the said polymer follows the (III) CH2=C(R1)-COO-[(EO)n(PO)n'(BO)n"]-Z in which R1 represents H or CH₃.

6. Composition according to any of claims 1 to 5, **characterized by** the fact that, the said polymer contains, compared with the total weight of the polymer:
- from 10 to 50 wt. % of monomers A,
- from 40 to 80 wt. % of monomers B,
- from 0.05 to 15 wt. % of monomers C, and
- from 0.05 to 10 wt. % of monomers D.

7. Composition according to any of claims 1 to 6, **characterized by** the fact, that the proportion of the agent varies from 0.1 to 20 wt. % compared with the total weight of the composition.
